# EUROPÄISCHE PATENTSCHRIFT

(11) **EP 3 277 212 B1**
(45) Veröffentlichungstag und Bekanntmachung des Hinweises auf die Patenterteilung: **01.05.2024**
(21) Anmeldenummer: 16708639.6
(22) Anmeldetag: 04.03.2016
(51) Int. Cl.: A61B 18/04

(54) **PLASMACHIRURGISCHE VORRICHTUNG SOWIE COMPUTERPROGRAMMPRODUKT ZUM BETREIBEN EINER SOLCHEN VORRICHTUNG**
PLASMA-SURGICAL DEVICE AND COMPUTER PROGRAM PRODUCT FOR OPERATING A DEVICE OF THIS TYPE
DISPOSITIF CHIRURGICAL AU PLASMA ET PRODUIT DE PROGRAMME INFORMATIQUE POUR FAIRE FONCTIONNER UN TEL DISPOSITIF

(30) Priorität: 30.03.2015 DE 102015205729
(43) Veröffentlichungstag der Anmeldung: 07.02.2018
(73) Patentinhaber: Olympus Winter & Ibe GmbH, 22045 Hamburg (DE)
(72) Erfinder: FAEHSING, Thomas, 38889 Blankenburg (DE)
(74) Vertreter: Seemann & Partner Patentanwälte mbB
(86) Internationale Anmeldenummer: PCT/EP2016/054641
(87) Internationale Veröffentlichungsnummer: WO 2016/155973

(56) Entgegenhaltungen:
- DE-A1-102014 003 382
- US-A1- 2008 097 425
- US-A1- 2012 022 522
- US-A1- 2014 276 725

## Beschreibung

Die Erfindung betrifft eine plasmachirurgische Vorrichtung, umfassend einen HF-Generator zum Erzeugen eines HF-Ansteuersignals, eine Gasquelle zum Bereitstellen eines Plasmagases und einen Plasma-Applikator mit einem Kanal, der an einem distalen Ende des Applikators ausmündet und der von dem Plasmagas durchströmbar ist oder durchströmt wird, und mit einer bevorzugt im Kanal angeordneten HF-Elektrode, die mit dem HF-Generator elektrisch verbunden ist, wobei die HF-Elektrode mit dem HF-Ansteuersignal beaufschlagbar ist oder beaufschlagt wird, so dass ausgehend vom distalen Ende des Applikators ein Plasma des aus dem Kanal ausströmbaren oder ausströmenden Plasmagases bereitstellbar ist oder bereitgestellt wird. Ferner betrifft die Erfindung ein Verfahren zum Betreiben einer solchen plasmachirurgischen Vorrichtung. Schließlich betrifft die Erfindung ein Computerprogrammprodukt.

Plasmachirurgische Vorrichtungen, beispielsweise Argon-Plasma-Koagulationsvorrichtungen, werden in der endoskopisch arbeitenden Medizin zum Stillen flächiger Blutungen eingesetzt. Ein weiteres Anwendungsgebiet plasmachirurgischer Vorrichtungen ist das großflächige Abtragen von Gewebe.

Plasmachirurgische Vorrichtungen basieren auf der thermischen Wirkung eines Hochfrequenzstroms, der über ein elektrisch ionisiertes Plasma, in vielen Fällen ein Argon-Plasma, auf das Gewebe appliziert wird. Zum Erzeugen des Plasmas sind eine Plasmagasquelle und eine Hochfrequenzquelle vorgesehen. Eine HF-Elektrode in einem Applikator wird mit einer hochfrequenten Wechselspannung beaufschlagt, so dass sich bei ausreichend hoher HF-Spannung und genügend kleinem Abstand zum Gewebe zwischen diesem und einem distalen Ende des Applikators das Plasma bildet. Dieses wird stabil aufrechterhalten, in dem permanent Plasmagas an einem distalen Ende des Applikators austritt.

Das ionisierte Plasma ist elektrisch leitfähig, so dass ausgehend von dem Applikator ein elektrischer Strom in das Gewebe appliziert wird. Der durch das Plasma geleitete Strom, der auf die Gewebeoberfläche auftrifft, bewirkt einen thermischen Effekt und führt so zu einer Blutstillung bzw. Koagulation.

Die Eindringtiefe ist begrenzt, so dass tieferliegende Gewebeschichten nicht mit erfasst werden. Dies schont das Gewebe und verringert das Perforationsrisiko. Da sich das Plasma außerdem nicht gradlinig, sondern entsprechend der elektrischen Feldlinien zwischen Gewebe und Applikator ausbreitet, werden auch schlecht zugängliche Bereiche erfasst. Da koaguliertes Gewebe eine geringere elektrische Leitfähigkeit als nicht koaguliertes Gewebe aufweist, wird die elektrische Energie bevorzugt in solche Bereiche eingetragen, in denen sie benötigt wird, um eine Blutstillung zu bewirken.

Aus der US 2014/0276725 A1 ist ein elektrochirurgisches System mit einem Steuergerät und einem Behandlungsstab bekannt. Der Behandlungsstab ist über ein elektrisches Kabel mit dem Steuergerät verbunden. Dem Behandlungsstab wird über einen Schlauch eine leitfähige Flüssigkeit, beispielsweise eine Kochsalzlösung, zugeführt, die am distalen Ende des Behandlungsstabs austritt. Am distalen Ende des Behandlungsstabs sind Elektroden vorhanden, die mit HF-Spannung beaufschlagt werden. So wird aus der am distalen Ende austretenden Kochsalzlösung ein Plasma geformt.

Aus der DE 10 2014 003 382 A1 ist ein elektrochirurgisches System mit einem Behandlungsstab bekannt, der an eine Steuerung angeschlossen ist. Zum Absaugen von überschüssigen Flüssigkeiten, Gewebefragmenten und/oder Ablationsprodukten aus dem Behandlungsvolumen erstreckt sich innerhalb des Schafts des Behandlungsstabs ein Sauglumen, welches über den Schlauch mit einer Peristaltikpumpe in Verbindung steht.

Es ist eine Aufgabe der Erfindung, eine plasmachirurgische Vorrichtung, ein Verfahren zum Betreiben einer plasmachirurgischen Vorrichtung sowie ein Computerprogrammprodukt anzugeben, wobei eine Stabilität des Plasmas verbessert werden soll. Die Erfindung wird durch den Schutzumfang der unabhängigen Ansprüche 1 und 5 eingeschränkt. Weitere Ausführungsformen sind in den abhängigen Ansprüchen offenbart.

Die Aufgabe wird gelöst durch eine plasmachirurgische Vorrichtung, umfassend einen HF-Generator zum Erzeugen eines HF-Ansteuersignals, eine Gasquelle zum Bereitstellen eines Plasmagases und einen Plasma-Applikator mit einem Kanal, der an einem distalen Ende des Applikators ausmündet und der von dem Plasmagas durchströmbar ist oder durchströmt wird, und mit einer bevorzugt im Kanal angeordneten HF-Elektrode, die mit dem HF-Generator elektrisch verbunden ist, wobei die HF-Elektrode mit dem HF-Ansteuersignal beaufschlagbar ist oder beaufschlagt wird, so dass ausgehend vom distalen Ende des Applikators ein Plasma des aus dem Kanal ausströmbaren oder ausströmenden Plasmagases bereitstellbar ist oder bereitgestellt wird, wobei die plasmachirurgische Vorrichtung dadurch fortgebildet ist, dass eine Steuereinheit und ein Durchflussregler zum Regeln einer von der Gasquelle in dem Kanal bereitgestellten Durchflussrate an Plasmagas umfasst sind, wobei die Steuereinheit dazu eingerichtet ist, eine Betriebsvariable des HF-Generators zu empfangen oder abzufragen und entsprechend einem hinterlegten Funktionszusammenhang den Durchflussregler so zu steuern, dass die Durchflussrate des Plasmagases mit einem erfassten Wert der Betriebsvariablen korreliert ist, wobei die Betriebsvariable eine DC-Offset-Spannung des HF-Generators, eine Amplitude oder ein Effektivwert eines HF-Stroms und/oder einer HF-Spannung des HF-Ansteuersignals ist.

Die plasmachirurgische Vorrichtung beruht auf den folgenden Erkenntnissen. Es ist derzeit vielfach üblich, die Durchflussrate des Plasmagases vor Beginn der Behandlung fest einzustellen und während der Behandlung auf einem konstanten Niveau zu belassen. Die Plasmagas-Flussrate ist also ungeachtet der Entfernung des Instruments bzw. des Applikators zum behandelnden Gewebe unverändert konstant. Es wurde erkannt, dass bei konstanter Plasmagas-Flussrate das Koagulationsergebnis vom Abstand des Applikators zum Gewebe abhängt. Mit anderen Worten sind das Plasma und dessen Qualität von der Plasmagassättigung zwischen der distalen Spitze des Applikators und dem Gewebe abhängig. Bei großem Abstand zwischen der distalen Spitze des Applikators und dem Gewebe kann die Plasmagaskonzentration in Gewebenähe zu gering für ein ausreichend stabiles Plasma sein. Die mögliche Folge ist ein Plasmaabriss gefolgt von einem automatisch durchgeführten anschließenden Zündversuch.

Ausgehend von diesen Erkenntnissen wird erfindungsgemäß vorgeschlagen, die Flussrate des Plasmagases an den Abstand des Applikators zum Gewebe anzupassen. Dies schließt sowohl die erstmalige Einstellung der Flussrate als auch ihre dynamische Regelung bzw. Steuerung ein. Es wurde ferner erkannt, dass als Maß oder Hinweis auf den Abstand zwischen dem distalen Ende des Applikators und dem Gewebe eine Betriebsvariable des HF-Generators ausgewertet werden kann. Die Flussrate des Plasmagases wird in Abhängigkeit von diesem Parameter verändert.

Vorteilhaft ist somit vorgesehen, dass die Flussrate bei großem Abstand zwischen Applikator und Gewebe größer ist als bei kleinem Abstand. So wird ein Abreißen des Plasmas verhindert, indem stets eine hinreichend große Konzentration des Plasmagases bereitgestellt wird. Gleichzeitig wird der Verbrauch an Plasmagas optimiert, da bei geringem Abstand lediglich ein geringer Fluss des Plasmagases eingestellt wird. Gegenüber herkömmlichen Lösungen, welche bei diesem Betriebszustand zu viel Plasmagas bereitstellen, wird Plasmagas eingespart, was zu Kostenvorteilen führt.

Bei dem Plasmagas handelt es sich beispielsweise um Argon.

Die plasmachirurgische Vorrichtung ist bevorzugt dadurch weitergebildet, dass der Fluss des Plasmagases automatisch auf der Basis einer vom Benutzer gewählten Wirkung, beispielsweise einer Koagulationswirkung, voreingestellt bzw. geregelt wird. Die Größe der gewählten Wirkung, welche beispielsweise in beliebigen Einheiten eingestellt oder vorgegeben wird, wird in elektrische Betriebsparameter des HF-Generators umgesetzt bzw. übersetzt. Die Einstellung des Plasmagas-Flusses erfolgt anschließend automatisch anhand des hinterlegten Funktionszusammenhangs auf der Grundlage eines erfassten Werts einer Betriebsvariablen des HF-Generators. Dabei ist insbesondere vorgesehen, dass der Benutzer die automatische Voreinstellung auf Wunsch beeinflussen kann, z.B. durch Anpassung eines Offsets oder einer Steigung des hinterlegten Funktionszusammenhangs. Hierfür wird dem Benutzer eine geeignete Bedienbarkeit bzw. eine geeignete Benutzerschnittstelle zur Verfügung gestellt.

Gemäß einer vorteilhaften Weiterbildung der plasmachirurgischen Vorrichtung ist ferner vorgesehen, dass die Betriebsvariable zeitlich veränderlich ist und die Steuereinheit dazu eingerichtet ist, fortlaufend mit der Zeit die Durchflussrate entsprechend dem hinterlegten Funktionszusammenhang zu verändern. Mit anderen Worten wird der Fluss des Plasmagases also nicht nur erstmalig in Abhängigkeit von der Betriebsvariablen des HF-Generators eingestellt, sondern auch zeitabhängig nachgeregelt. So ist vorteilhaft sichergestellt, dass zu jedem Zeitpunkt eine ausreichend hohe Plasmagaskonzentration vorliegt. Es wird sowohl einer Über- als auch einer Unterversorgung mit Plasmagas vorgebeugt.

In einer weiteren vorteilhaften Ausführungsform ist vorgesehen, dass entsprechend dem Funktionszusammenhang mit steigendem Wert der Betriebsvariablen die Durchflussrate des Plasmagases steigt und ferner insbesondere mit fallendem Wert der Betriebsvariablen die Durchflussrate des Plasmagases sinkt. Insbesondere ist ein proportionaler Funktionszusammenhang zwischen dem Wert der Betriebsvariablen und der Durchflussrate vorgesehen.

Entsprechend dem hinterlegten Funktionszusammenhang steigt der Fluss des Plasmagases mit zunehmender Entfernung des Applikators beispielsweise von einer Gewebswand. Selbstverständlich sinkt der Wert des Plasmagasflusses mit fallendem Abstand zur Gewebewand. Ein proportionaler Funktionszusammenhang zwischen dem Wert der Betriebsvariablen und der Durchflussrate hat sich als besonders vorteilhaft erwiesen. Selbstverständlich sind auch weitere Funktionszusammenhänge vorgesehen, welche beispielsweise den Plasmagasfluss bei geringen Abständen überproportional verringern und/oder bei großen Abständen überproportional erhöhen.

Die Betriebsvariable ist eine DC-Offset-Spannung des HF-Generators, eine Amplitude oder ein Effektivwert eines HF-Stroms und/oder einer HF-Spannung des HF-Ansteuersignals. Mit anderen Worten handelt es sich bei dem HF-Strom und der HF-Spannung um einen Ausgangsstrom bzw. eine Ausgangsspannung des HF-Generators. Im Betrieb der plasmachirurgischen Vorrichtung wird eine HF-Spannung zwischen der HF-Elektrode und einer mit dem Gewebe in Kontakt stehenden Elektrode angelegt. In dem zwischen dem Applikator und der Gewebswand ausgebildeten Plasma fließt ein elektrischer Strom. Diese Plasmaentladung verursacht in dem angelegten HF-Ansteuersignal einen DC-Offset, dessen Wert im HF-Generator vorliegt. Ebenso liegen die Werte für die Amplitude oder den Effektivwert des fließenden HF-Stroms und/oder der angelegten HF-Spannung (des HF-Ansteuersignals) im HF-Generator vor.

Es wurde ferner erkannt, dass die Werte der genannten elektrischen Größen, beispielsweise ein Wert der DC-Offset-Spannung, ein Maß für die Entfernung des Applikators von beispielsweise der Gewebswand ist. Somit ist die Regelung der Durchflussrate des Plasmagases in Abhängigkeit von einer der genannten Größen besonders effektiv und einfach.

Genauer gesagt wird im Betrieb der plasmachirurgischen Vorrichtung eine Ausgangsstufe des HF-Generators so geregelt, dass je nach gewünschtem therapeutischen Effekt entweder eine DC-Offset-Spannung oder die Amplitude oder der Effektivwert der angelegten HF-Spannung oder des HF-Stroms auf bzw. nahe bei einem voreingestellten Sollwert gehalten werden. Dazu werden einige oder auch alle anderen Betriebsparameter im Sinne einer Regelung nachgeführt. Es wurde ferner erkannt, dass jeweils ein nachgeführter Betriebsparameter ein Maß für die Entfernung des Applikators beispielsweise von der Gewebswand sein kann. Somit ist die Regelung der Durchflussrate des Plasmagases in Abhängigkeit von dem jeweiligen nachgeführten Betriebsparameter besonders effektiv und einfach.

Gemäß einer weiteren vorteilhaften Ausführungsform ist vorgesehen, dass die plasmachirurgische Vorrichtung eine Plasma-Koagulationsvorrichtung ist.

Die Aufgabe wird ferner gelöst durch ein nicht beanspruchtes Verfahren zum Betreiben einer plasmachirurgischen Vorrichtung gemäß einer oder mehreren der genannten Ausführungsformen. Bei einem solchen Verfahren ist vorgesehen, dass von dem HF-Generator ein HF-Ansteuersignal erzeugt und von der Gasquelle das Plasmagas bereitgestellt wird, wobei der Kanal des Applikators von dem Plasmagas durchströmt und die Elektrode mit dem HF-Ansteuersignal beaufschlagt wird, so dass am distalen Ende des Applikators das Plasma bereitgestellt wird, wobei die Steuereinheit die Betriebsvariable des HF-Generators empfängt oder abfragt und entsprechend dem hinterlegten Funktionszusammenhang den Durchflussregler so steuert, dass die Durchflussrate des Plasmagases mit dem erfassten Wert der Betriebsvariablen korreliert ist.

Gleiche oder ähnliche Vorteile, wie sie bereits im Hinblick auf die plasmachirurgische Vorrichtung erwähnt wurden, treffen auch auf das Verfahren zum Betreiben derselben zu und sollen daher nicht wiederholt werden.

Gemäß einer vorteilhaften Ausführungsform ist das nicht beanspruchte Verfahren dadurch fortgebildet, dass die Betriebsvariable zeitlich veränderlich ist und die Steuereinheit fortlaufend mit der Zeit die Durchflussrate entsprechend dem hinterlegten Funktionszusammenhang ändert.

Gemäß einer weiteren vorteilhaften Ausführungsform ist vorgesehen, dass mit steigendem Wert der Betriebsvariablen die Durchflussrate des Plasmagases erhöht wird, wobei insbesondere die Durchflussrate des Plasmagases in Abhängigkeit vom Wert der Betriebsvariablen proportional verändert wird.

Schließlich ist das nicht beanspruchte Verfahren zum Betreiben der plasmachirurgischen Vorrichtung vorteilhaft dadurch fortgebildet, dass als Betriebsvariable eine DC-Offset-Spannung des HF-Generators, eine Amplitude oder ein Effektivwert eines HF-Stroms und/oder einer HF-Spannung des HF-Ansteuersignals empfangen oder abgefragt wird.

Die erfindungsgemäße Aufgabe wird ferner gelöst durch ein Computerprogrammprodukt, welches eine plasmachirurgische Vorrichtung gemäß einer oder mehreren der genannten Ausführungsformen dazu veranlasst, ein Verfahren nach einer oder mehreren der zuvor genannten Ausführungsformen durchzuführen. Auch auf das Computerprogrammprodukt treffen gleiche oder ähnliche Vorteile zu, wie sie bereits im Hinblick auf die plasmachirurgische Vorrichtung erwähnt wurden.

Weitere Merkmale der Erfindung werden aus der Beschreibung erfindungsgemäßer Ausführungsformen zusammen mit den Ansprüchen und der beigefügten Zeichnung ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt:

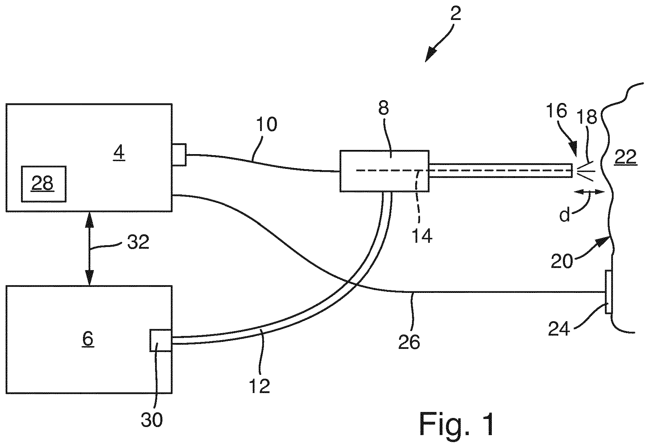

- Fig. 1: eine plasmachirurgisches Vorrichtung in schematisch vereinfachter Darstellung.

Fig. 1 zeigt eine plasmachirurgische Vorrichtung 2 in schematisch vereinfachter Darstellung. Diese umfasst einen HF-Generator 4 zum Erzeugen eines HF-Ansteuersignals. Ferner ist eine Gasquelle 6 vorhanden, welche ein Plasmagas, beispielsweise Argon, bereitstellt. Ein Plasma-Applikator 8 ist sowohl mit dem HF-Generator 4 über ein geeignetes HF-Verbindungskabel 10 als auch mit der Gasquelle 6 über einen geeigneten Gas-Verbindungsschlauch 12 verbunden.

Im Innenraum des Applikators 8 befindet sich ein schematisch mit gestrichelter Linie angedeuteter Kanal 14, der an einem distalen Ende 16 des Applikators 8 ausmündet. Der Kanal 14 ist von dem Plasmagas, welches dem Plasma-Applikator 8 von der Gasquelle 6 zugeführt wird, durchströmbar bzw. im Betrieb der plasmachirurgischen Vorrichtung 2 durchströmt. In dem Kanal 14 des Plasma-Applikators 8 ist eine nicht dargestellte HF-Elektrode angeordnet, die mit dem HF-Generator 4 elektrisch verbunden ist. Diese HF-Elektrode wird mit einem HF-Ansteuersignal beaufschlagt, welches über das HF-Verbindungskabel 10 von dem HF-Generator 4 an den Plasma-Applikator 8 übertragen wird.

Das in dem Kanal 14 vorhandene Plasmagas wird ionisiert, so dass am distalen Ende 16 des Applikators 8 ein Plasma 18 bereitgestellt wird. Das Plasma 18 wird auf eine Oberfläche 20 eines Gewebes 22 appliziert. Das Gewebe 22 ist beispielsweise menschliches oder tierisches Gewebe eines Organs, bei der Oberfläche 22 handelt es sich entsprechend beispielsweise um eine Organwand.

Ausgehend von dem Plasma-Applikator 8 wird über das ionisierte und damit elektrisch leitfähige Plasma 18 elektrische Energie auf die Oberfläche 20 des Gewebes 22 übertragen. Dort bewirkt die elektrische Energie eine lokale Erwärmung des Gewebes 22, die beispielsweise eine Koagulation, d.h. eine Blutstillung, auf der Oberfläche 20 bewirkt. Mit anderen Worten handelt es sich bei der dargestellten plasmachirurgischen Vorrichtung 2 bevorzugt um eine Plasma-Koagulationsvorrichtung, insbesondere um eine Argon-Plasma-Koagulationsvorrichtung.

Aufgrund der geringen Eindringtiefe der über das Plasma 18 übertragenen thermischen Energie werden tieferliegende Gewebeschichten nicht miterfasst und geschont, so dass aufgrund der geringen Eindringtiefe ein ebenfalls geringes Perforationsrisiko für die Oberfläche 20 besteht. Die elektrische Leitfähigkeit von bereits koaguliertem Gewebe ist außerdem geringer als diejenige von nicht koaguliertem Gewebe, so dass bevorzugt dort ein Energieeintrag stattfindet, wo noch lokale Blutungen zu stillen sind.

Die plasmachirurgische Vorrichtung 2 umfasst ferner eine Neutralelektrode 24, welche, sofern es sich um eine monopolare Vorrichtung handelt, großflächig in Kontakt mit dem zu behandelnden Patienten steht. Ferner ist vorgesehen, dass die Neutralelektrode 24 direkt im oder am Gewebe 22 (so wie in Fig. 1 angedeutet) platziert wird, an welchem die Blutung zu stillen ist. Über eine entsprechende Rückleitung 26 ist die Neutralelektrode 24 mit dem HF-Generator 4 gekoppelt.

Die plasmachirurgische Vorrichtung 2 umfasst ferner eine Steuereinheit 28, welche lediglich beispielhaft als Teil des HF-Generators 4 dargestellt ist. Ferner ist ein Durchflussregler 30 vorhanden.

Der Durchflussregler 30 regelt eine von der Gasquelle 6 in dem Kanal 14 des Plasma-Applikators 8 bereitgestellte Durchflussrate des Plasmagases. Die Steuereinheit 28 ist dazu eingerichtet, eine Betriebsvariable des HF-Generators 4 zu empfangen oder abzufragen. Bei dieser Betriebsvariablen handelt es sich beispielsweise um eine DC-Offset-Spannung des HF-Generators 4.

In der Steuereinheit 28 ist ein Funktionszusammenhang hinterlegt. Mit anderen Worten ist die Steuereinheit 28, bei welcher es sich beispielsweise um einen Mikrocontroller aber auch um einen Computer oder eine Workstation handelt, mit entsprechenden Programmmitteln versehen, welche den beschriebenen Funktionszusammenhang bereitstellen. Wenn die Steuereinheit 28 ein Computer oder eine Workstation ist, ist sie bevorzugt außerhalb des HF-Generators 4 vorhandenen.

Die Steuereinheit 28 ist ferner dazu eingerichtet, den Durchflussregler 30 entsprechend dem hinterlegten Funktionszusammenhang so zu steuern, dass die Durchflussrate des Plasmagases im Kanal 14 mit dem erfassten Wert der Betriebsvariablen des HF-Generators 4 korreliert ist. Zu diesem Zweck sind der HF-Generator 4 und die Gasquelle 6 über eine geeignete Datenverbindung 32 miteinander gekoppelt.

Die Steuereinheit 28 ist gemäß einem weiteren Ausführungsbeispiel dazu eingerichtet, die Durchflussrate des Plasmagases in dem Kanal 14 nicht nur zu steuern, sondern auch rückkoppelnd zu regeln. Zu diesem Zweck ist ein nicht dargestellter Massestrommesser (flow controller) in dem Kanal 14 vorgesehen.

Die plasmachirurgische Vorrichtung 2 ist bevorzugt dazu eingerichtet, dass die Betriebsvariable, also beispielsweise die DC-Offset-Spannung des HF-Generators 4, zeitlich veränderlich ist und auch als zeitlich veränderliche Größe von der Steuereinheit 28 erfasst bzw. abgefragt wird. Die Steuereinheit 28 ist entsprechend dazu eingerichtet, fortlaufend mit der Zeit die Durchflussrate des Plasmagases in dem Kanal 14 entsprechend dem hinterlegten Funktionszusammenhang zu verändern bzw. nachzuregeln oder anzupassen.

Es ist also mit anderen Worten vorgesehen, dass die Steuereinheit 28 die benötigte Durchflussrate des Plasmagases in dem Kanal 14 sowohl erstmalig bei Inbetriebnahme der plasmachirurgischen Vorrichtung 2 einstellt, als auch in Betrieb dynamisch nachregelt. So wird vorteilhaft die Möglichkeit geschaffen, bei sich veränderndem Abstand d zwischen dem distalen Ende 16 des Plasma-Applikators 8 und der Oberfläche 20 des Gewebes 22 den Fluss des Plasmagases im Kanal 14 anzupassen.

Während bei einem geringen Abstand d ein geringer Fluss des Plasmagases ausreicht, um das Plasma 18 aufrechtzuerhalten, ist bei steigendem Abstand d eine zunehmende Menge Plasmagas notwendig, um ein Abreißen des Plasmas 18 zu verhindern. So ist vorteilhaft sichergestellt, dass das Plasma 18 auch bei veränderlichem Abstand d zwischen der Oberfläche 20 des Gewebes 22 und dem distalen Ende 16 des Plasma-Applikators 8 zuverlässig aufrechterhalten wird. Gleichzeitig wird die Menge des eingesetzten Plasmagases optimiert, so dass nicht unnötig viel Plasmagas eingesetzt wird. Dies verbessert die Wirtschaftlichkeit der plasmachirurgischen Vorrichtung 2.

Der in der Steuereinheit 28 hinterlegte Funktionszusammenhang steuert die oben beschriebene Funktionalität zwischen dem Fluss des Plasmagases in dem Kanal 14 des Plasma-Applikators 8 und dem Abstand d. Zu diesem Zweck fragt die Steuereinheit 28 fortlaufend beispielsweise eine DC-Offset-Spannung des HF-Generators 4 ab und steuert den Durchflussregler 30 der Gasquelle 6 über die Datenverbindung 32 entsprechend dem hinterlegten Funktionszusammenhang zwischen der Betriebsvariablen des HF-Generators 4 (DC-Offset-Spannung) und der Durchflussrate des Plasmagases. Als besonders vorteilhaft hat sich ein proportionaler Funktionszusammenhang zwischen dem Wert der Betriebsvariablen und der Durchflussrate erwiesen.

Gemäß einem Verfahren zum Betreiben der plasmachirurgischen Vorrichtung 2 gemäß einem oder mehreren der genannten erfindungsgemäßen Aspekte wird von dem HF-Generator 4 ein HF-Ansteuersignal erzeugt und von der Gasquelle 6 das Plasmagas bereitgestellt. Der Kanal 14 des Plasma-Applikators 8 wird von Plasmagas durchströmt und die Elektrode (nicht dargestellt) wird mit dem HF-Ansteuersignal beaufschlagt. Am distalen Ende 16 des Applikators 8 wird das Plasma 18 bereitgestellt. Die Steuereinheit 28, die die Betriebsvariable des HF-Generators 4 empfängt oder abfragt, steuert entsprechend dem hinterlegten Funktionszusammenhang den Durchflussregler 30 so, dass die Durchflussrate des Plasmagases in dem Kanal 14 mit dem erfassten Wert der Betriebsvariablen korreliert ist.

Diese Steuerung erfolgt insbesondere zeitlich fortlaufend entsprechend dem in der Steuereinheit 28 hinterlegten Funktionszusammenhang. Dabei ist ferner bevorzugt vorgesehen, dass mit steigendem Wert der Betriebsvariablen, also beispielsweise mit steigender DC-Offset-Spannung, welche einen steigenden Abstand d zwischen dem distalen Ende 16 des Plasma-Applikators 8 und der Oberfläche 20 des Gewebes 22 anzeigt, auch der Fluss des Plasmagases erhöht wird. Gleiches gilt auch umgekehrt, so wird mit geringer werdendem Abstand d der Fluss des Plasmagases verringert.

Bevorzugt ist die Steuereinheit 28 eine Recheneinheit, welche die allgemein bekannten Komponenten aufweist. Beispielsweise umfasst die Steuereinheit 28, bei welcher es sich beispielsweise um einen PC, eine Workstation oder einen Mikrocontroller handelt, einen nicht flüchtigen Speicher, in dem unter anderem der Funktionszusammenhang hinterlegt ist.

Ferner ist die Steuereinheit 28 dazu eingerichtet ein Computerprogrammprodukt auszuführen, welches die plasmachirurgische Vorrichtung 2 dazu veranlasst, das zuvor erläuterte Verfahren durchzuführen. Auch das Computerprogramm ist in dem nicht flüchtigen Speicher der Steuereinheit 28 abgelegt.

Alle genannten Merkmale, auch die der Zeichnung allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: plasmachirurgische Vorrichtung
- 4: HF-Generator
- 6: Gasquelle
- 8: Plasma-Applikator
- 10: HF-Kabel
- 12: Gas-Verbindungsschlauch
- 14: Kanal
- 16: distales Ende
- 18: Plasma
- 20: Oberfläche
- 22: Gewebe
- 24: Neutralelektrode
- 26: Rückleitung
- 28: Steuereinheit
- 30: Durchflussregler
- 32: Datenverbindung

- d: Abstand

## Patentansprüche

1. Plasmachirurgische Vorrichtung (2), umfassend einen HF-Generator (4) zum Erzeugen eines HF-Ansteuersignals, eine Gasquelle (6) zum Bereitstellen eines Plasmagases und einen Plasma-Applikator (8) mit einem Kanal (14), der an einem distalen Ende (16) des Applikators (8) ausmündet und der von dem Plasmagas durchströmbar ist oder durchströmt wird, und mit einer bevorzugt im Kanal (14) angeordneten HF-Elektrode, die mit dem HF-Generator (4) elektrisch verbunden ist, wobei die HF-Elektrode mit dem HF-Ansteuersignal beaufschlagbar ist oder beaufschlagt wird, so dass ausgehend vom distalen Ende (16) des Applikators (8) ein Plasma (18) des aus dem Kanal (14) ausströmbaren oder ausströmenden Plasmagases bereitstellbar ist oder bereitgestellt wird, wobei eine Steuereinheit (28) und ein Durchflussregler (30) zum Regeln einer von der Gasquelle (6) in dem Kanal (14) bereitgestellten Durchflussrate an Plasmagas umfasst sind,
**dadurch gekennzeichnet, dass** die Steuereinheit (28) dazu eingerichtet ist, eine Betriebsvariable des HF-Generators (4) zu empfangen oder abzufragen und entsprechend einem hinterlegten Funktionszusammenhang den Durchflussregler (30) so zu steuern, dass die Durchflussrate des Plasmagases mit einem erfassten Wert der Betriebsvariablen korreliert ist, wobei die Betriebsvariable eine DC-Offset-Spannung des HF-Generators (4), eine Amplitude oder ein Effektivwert eines HF-Stroms und/oder einer HF-Spannung des HF-Ansteuersignals ist.

2. Vorrichtung (2) nach Anspruch 1, **dadurch gekennzeichnet, dass** die Betriebsvariable zeitlich veränderlich ist und die Steuereinheit (28) dazu eingerichtet ist, fortlaufend mit der Zeit die Durchflussrate entsprechend dem hinterlegten Funktionszusammenhang zu verändern.

3. Vorrichtung (2) nach Anspruch 1 oder 2, **dadurch gekennzeichnet, dass** entsprechend dem Funktionszusammenhang mit steigendem Wert der Betriebsvariablen die Durchflussrate des Plasmagases steigt, wobei insbesondere ein proportionaler Funktionszusammenhang zwischen dem Wert der Betriebsvariablen und der Durchflussrate vorgesehen ist.

4. Vorrichtung (2) nach einem der Ansprüche 1 bis 3, **dadurch gekennzeichnet, dass** die plasmachirurgische Vorrichtung eine Plasma-Koagulationsvorrichtung ist.

5. Computerprogrammprodukt, welches eine plasmachirurgische Vorrichtung (2) nach einem der Ansprüche 1 bis 4 dazu veranlasst, ein Verfahren zum Betreiben der plasmachirurgischen Vorrichtung (2) durchzuführen, bei dem von dem HF-Generator (4) das HF-Ansteuersignal erzeugt und von der Gasquelle (6) das Plasmagas bereitgestellt wird, wobei der Kanal (14) des Plasma-Applikators (8) von dem Plasmagas durchströmt und die Elektrode mit dem HF-Ansteuersignal beaufschlagt wird, so dass am distalen Ende (16) des Applikators (8) das Plasma (18) bereitgestellt wird, wobei die Steuereinheit (28) die Betriebsvariable des HF-Generators (4) empfängt oder abfragt und entsprechend dem hinterlegten Funktionszusammenhang den Durchflussregler (30) so steuert, dass die Durchflussrate des Plasmagases mit dem erfassten Wert der Betriebsvariablen korreliert ist, wobei als die Betriebsvariable die DC-Offset-Spannung des HF-Generators (4), die Amplitude oder der Effektivwert des HF-Stroms und/oder der HF-Spannung des HF-Ansteuersignals empfangen oder abgefragt wird.

## Claims

1. A plasma surgery apparatus (2), comprising an HF-generator (4) for generating an HF-control signal, a gas source (6) for providing a plasma gas, and a plasma applicator (8) having a channel (14) which opens out at a distal end (16) of the applicator (8) and through which the plasma gas flows or can flow, and having an HF-electrode that is preferably arranged in the channel (14) and is electrically connected to the HF-generator (4), wherein the HF-electrode is or can be supplied with the HF-control signal in that, originating from the distal end (16) of the applicator (8), a plasma (8) of the plasma gas that flows or can flow from the channel (14) is or can be provided, wherein it comprises a control unit (28) and a flow controller (30) for controlling a flow rate of plasma gas provided by the gas source (6) in the channel (14), **characterized in that** the control unit (28) is configured to receive or request an operating variable of the HF-generator (4) and, according to a stored functional relationship, to control the flow controller (30) **in that** the flow rate of the plasma gas is correlated with a detected value of the operating variable, wherein the operating variable is a DC-offset-voltage of the HF-generator (4), an amplitude or an effective value of an HF-current, and/or an HF-voltage of the HF-control signal.

2. The apparatus (2) according to claim 1, **characterized in that** the operating variable changes over time, and the control unit (28) is configured to continuously change the flow rate over time corresponding to the stored functional relationship.

3. The apparatus (2) according to claim 1 or 2, **characterized in that** the flow rate of the plasma gas increases corresponding to the functional relationship as the value of the operating variable increases, wherein, in particular, a proportional functional relationship is provided between the value of the operating variable and the flow rate.

4. The apparatus (2) according to one of claims 1 to 3, **characterized in that** the plasma surgery apparatus is a plasma coagulation apparatus.

5. A computer program product that causes a plasma surgery apparatus (2) according to one of claims 1 to 4 to execute a method for operating a plasma surgery apparatus (2), wherein an HF-control signal is generated by the HF-generator (4) and the plasma gas is provided by the gas source (6), wherein the plasma gas flows through the channel (14) of the plasma applicator (8) and the HF control signal is applied to the electrode in that the plasma (18) is provided at the distal end (16) of the applicator (8), wherein the control unit (28) receives or queries the operating variable of the HF-generator (4) and controls the flow controller (30) corresponding to the stored functional relationship in that the flow rate of the plasma gas is correlated to the detected value of the operating variable, wherein the receiver or queried operating variable is a DC-offset-voltage of the HF-generator (4), an amplitude or an effective value of an HF-current, and/or an HF-voltage of the HF-control signal.

## Revendications

1. Dispositif chirurgical au plasma (2), comprenant un générateur HF (4) pour générer un signal de commande HF, une source de gaz (6) pour fournir un gaz plasma et un applicateur de plasma (8) avec un canal (14) qui débouche à une extrémité distale (16) de l'applicateur (8) et qui est apte à être traversé ou est traversé par le gaz plasma, et avec une électrode HF de préférence agencée dans le canal (14), qui est reliée électriquement au générateur HF (4), l'électrode HF étant apte à être alimentée ou étant alimentée par le signal de commande HF, de sorte qu'un plasma (18) du gaz plasma qui est apte à s'écouler ou qui s'écoule du canal (14) est apte à être fourni ou est fourni à partir de l'extrémité distale (16) de l'applicateur (8), une unité de commande (28) et un régulateur de débit (30) étant inclus, pour réguler un débit de gaz plasma fourni par la source de gaz (6) dans le canal (14),
**caractérisé en ce que** l'unité de commande (28) est conçue de façon à recevoir ou à interroger une variable de fonctionnement du générateur HF (4) et, conformément à une relation fonctionnelle mémorisée, pour commander le régulateur de débit (30) de telle sorte que le débit du gaz plasma soit corrélé à une valeur détectée de la variable de fonctionnement, la variable de fonctionnement étant une tension de décalage en CC du générateur HF (4), une amplitude ou une valeur efficace d'un courant HF et/ou d'une tension HF du signal de commande HF.

2. Dispositif (2) selon la revendication 1, **caractérisé en ce que** la variable de fonctionnement est variable dans le temps et **en ce que** l'unité de commande (28) est conçue de façon à faire varier le débit dans le temps, en continu, en fonction de la relation fonctionnelle mémorisée.

3. Dispositif (2) selon la revendication 1 ou la revendication 2, **caractérisé en ce que**, conformément à la relation fonctionnelle, le débit du gaz plasma augmente à mesure que la valeur de la variable de fonctionnement augmente, une relation fonctionnelle proportionnelle étant notamment prévue entre la valeur de la variable de fonctionnement et le débit.

4. Dispositif (2) selon l'une des revendications 1 à 3, **caractérisé en ce que** le dispositif de chirurgie au plasma est un dispositif de coagulation par plasma.

5. Produit programme d'ordinateur amenant un dispositif de chirurgie au plasma (2) selon l'une quelconque des revendications 1 à 4 à mettre en oeuvre un procédé de fonctionnement du dispositif de chirurgie au plasma (2), dans lequel le signal de commande HF est généré par le générateur HF (4) et le gaz plasma est fourni par la source de gaz (6), le canal (14) de l'applicateur de plasma (8) étant traversé par le gaz plasma et l'électrode étant alimentée par le signal de commande HF, de sorte que le plasma (18) est fourni à l'extrémité distale (16) de l'applicateur (8), l'unité de commande (28) recevant ou interrogeant la variable de fonctionnement du générateur HF (4) et commandant le régulateur de débit (30) en fonction de la relation fonctionnelle mémorisée de telle sorte que le débit du gaz plasma soit corrélé à la valeur détectée de la variable de fonctionnement, à la tension de décalage DC du générateur HF (4), l'amplitude ou à la valeur efficace du courant HF et/ou à la tension HF du signal de commande HF reçue ou interrogée en tant que variable de fonctionnement.
